# EUROPEAN PATENT APPLICATION

(11) **EP 2 752 238 A1**
(43) Date of publication of application: **09.07.2014**
(21) Application number: 11871792.5
(22) Date of filing: 01.09.2011
(51) Int. Cl.: B01J 21/04, C07C 1/20, C07C 2/54

(54) **METHOD FOR THE ALKYLATION OF RESIDUAL HYDROCARBONS FROM PYROLYTIC PROCESSES**

(71) Applicant: Guradoor, S.L., 38390 Santa Úrsula (Tenerife) (ES)
(72) Inventor: GONZÁLEZ GONZÁLEZ, Daniel, E-38390 Santa Ursula (Tenerife) (ES)
(74) Representative: Gil-Vega, Victor
(86) International application number: PCT/ES2011/070615
(87) International publication number: WO 2013/030413

(57) **Abstract**

The invention relates to a method for the alkylation of residual hydrocarbons obtained from pyrolytic processes, in particular processes for the obtaining of synthesis gas from wet crushed coal, essentially alkene- and alkane-type waste products, in order the exploit the energy thereof in the form of alkenes, alkanes and alcohols having a high energy content. The method is essentially characterised in that the waste products obtained from the synthesis gas formation are subject to a subsequent treatment in order to transform them into other branched alkane-type products and alcoholic compounds, recovering the hydrogen and water produced in said reactions, that remain available for their subsequent use as fuel in other chemical processing plants, or to be fed back to the gasification-pyrolysis process itself, to enrich the synthesis gas obtained.

## Description

The present invention generally relates to a method for the alkylation of residual hydrocarbons obtained from pyrolytic processes, and especially, to a process for the obtaining of synthesis gas, which essentially consists of a mixture of CO, CO₂ and H₂, from wet crushed coal.

Specifically, the present invention relates to a method for the alkylation of residual hydrocarbons obtained from pyrolytic processes, and especially, from a process intended for the obtaining of synthesis gas from wet crushed coal, essentially alkene- and alkane-type residues, so that their energy content may be exploited as isomeric and polymeric alcohols with a high energy content.

The reactions involved in the coal gasification process are essentially the following ones:
- C + ½O₂ →CO ΔHr = -9,25 MJ/Kg
- C + CO₂ →2CO ΔHr = 14,37 MJ/Kg
- C + H₂O →CO + H₂ ΔHr = 10,94 MJ/Kg
- CH₄ + ½O₂ →CO + 2H₂ ΔHr = -35,7 MJ/kmol
- H₂ + ½O₂ →H₂O ΔHr = -242 MJ/kmol
- CH₄ + H₂O →CO + 3H₂ ΔHr = 206 MJ/kmol
- CO + H₂O -CO₂ + H₂ ΔHr = -41,1 MJ/kmol

And to a lesser extent and, as secondary reactions,
- CO + ½O₂ →CO₂ + 67Kcal/mol
- CO + 3H₂→ CH₄ + H₂O + 49 Kcal/mol
- CO₂ + 4H₂→ CH₄ + 2 H₂O + 42 Kcal/mol

As the above-mentioned reactions are actually equilibriums, certain factors, such as temperature or pressure, may significantly alter the output of the gasification process and the nature of the waste products obtained, and basically, the gas obtained has a low percentage of methane, tars (paraffins and olefins) and oils.

As in the case of the processes for the alkylation of oil cracking derivatives, some of the minor alkanes and alkenes obtained as waste products of the coal gasification-pyrolysis reactions are converted into synthetic fuels with a high-octane rating using similar alkylation reactions.

Thus, the term alkylation refers to a process for the production of a component of high-octane gasolines, through the synthesis of butylenes with isobutane. The alkylation process consists of a chemical synthesis wherein a branched-chain alkane is linked to the double-bond of an alkene, which has been obtained from the cracking or second stage of oil distillation. The product obtained from such synthesis is called alkylate or alkylated gasoline, which is a product comprised by isoparaffinic components. The process is aimed at producing a fraction whose technical (high octane rating) and environmental (low vapour pressures and photochemical reactivity) currently make it one of the most important components of reformulated gasoline. Alkylation is a catalytic process which calls for a strong acid catalyst.

In the case of residual processes for the obtaining of synthesis gas through gasification-pyrolysis, the nature of the final product is influenced by the structure and composition of the naphtha fed, i.e., the nature of the residual hydrocarbon mixture thus obtained.

For instance, the alkylation comprises a reaction of isobutane, which can be previously obtained from a butane reforming reaction to obtain isobutane, which in turn can be subsequently alkylated to obtain isobutylene and other olefins.

The term alkylation is generally applied to catalysed reactions between isobutane and several light olefins. The product is a highly-branched saturated hydrocarbon used to increase the octane rating of gasoline. The alkylating reaction implies the addition of an H⁺ proton to a double bond of an olefin, to obtain a carbonium ion. The catalyst must be of the acid type, to foster the formation of cations (carbonium ion) and the most used ones are aluminium trichloride with hydrochloric acid, sulphuric acid and hydrofluoric acid.

Similarly, according to a reforming reaction, any alkane may be converted into another alkane of lower molecular weight and an olefin, as indicated in the following reaction, where n > x + y,

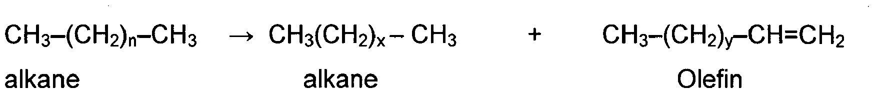

Therefore, waste products from the process for the obtaining of synthesis gas from crushed coal provide a source of by-products of the light olefin and branched alkanes type, that provide the necessary reagents for the subsequent reaction. The dehydrogenation of alkanes to obtain alkenes, for instance, eliminates Hydrogen gas through heating, according to a endothermic catalytic reaction, but which is favourable from an entropy perspective, where the heat source is originated at the coal gasification-pyrolysis stages and the by-product of the reaction is used as a fuel or at other processing plants, or it is simply recirculated towards the gasification reactor to enrich the synthesis gas thus obtained.

Similarly to the alkenes obtained from the cracking, essentially acetylene, the alkenes obtained as waste products from the process aimed at obtaining synthesis gas may be converted through hydration into alcohols with interest from a synthetic point of view. Since ethylene and water react during the gaseous stage (vapour) and the reaction is developed in that direction, decreasing the number of molecules present in the mixture, it stems that the displacing of the equilibrium in the direction of the alcohol formation contributes to the pressure increase. In this case, in order to achieve that the reaction is carried out at sufficient speed, the use of the catalyst and the heating of the substances are required. However, as the reaction is of an exothermic nature, an excessively strong heating will accelerate the reaction, which is developed with heat absorption, or in other words, the decomposition of the alcohol formed and the displacement of the equilibrium on the opposite direction. It has been established that the optimum conditions for ethylene hydration are a temperature range of 280 to 300°C and a pressure range of 7 to 8 Mpa, using the phosphoric acid deposited on a solid carrier as reaction catalyst. Under these conditions, around 5% of the initial ethylene is transformed into alcohol upon its passing through the contact device. Consequently, to yield the reaction profitable, it is necessary to separate the alcohol from the reaction products and recirculate the ethene for a new hydration, i.e., the circulation process must be implemented. It is also evident that the exhaust products of the reaction may be used to heat the substances arriving for hydration.

On the other hand, the dehydration of the alcohols obtained requires the presence of an acid and heat. Generally speaking, two different methods may be employed: (a) heating the alcohol with sulphuric or phosphoric acid, and (b) passing the vapour through a catalyst, preferably alumina (Al₂O₃), at high temperatures (Alumina works as an acid, as a Lewis acid or, through the intermediary of OH groups in its surface, as a Lowry-Bronsted acid).

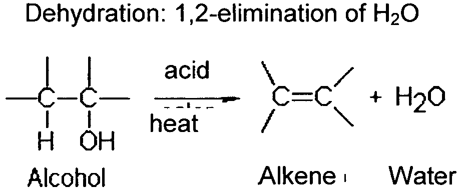

As opposed to the base-induced 1,2-elimination, dehydration is a reversible process.

In accordance with the above, each step of the method has a reversible nature. Under dehydration conditions, the alkene, which is quite volatile, is generally detached from the reaction mixture, and consequently, the equilibrium is displaced towards the right and the whole sequence of reactions is forced towards elimination.

Thus, the object of the invention is to provide a method for the alkylation and hydration/dehydration of residual hydrocarbons obtained from a process to obtain synthesis gas, and especially from a wet crushed coal gasification-pyrolysis process, wherein such waste products basically consist of gases of diverse composition which can be employed as fuels, in some cases, and as raw materials, in other cases.

The method described herein allows to alkylate the waste materials originated in a method for obtaining synthesis gas, especially from a wet crushed coal gasification-pyrolysis method, so that at least part of such waste material is transformed into compounds with a higher octane rating, such as branched alkanes, and/or used to hydrate/dehydrate the alkenes obtained, in order to obtain alcohols. As it has been already mentioned, since the final by-product released by some of the reactions of this process may by hydrogen or water, such hydrogen can be recycled to be subsequently used at chemical plants, or to be fed back to the gasification-pyrolysis process itself, to enrich the synthesis gas obtained.

To that effect, the method according to the invention comprises the submission of the waste products obtained from the formation of the synthesis gas from wet crushed coal, according to a gasification-pyrolysis process, to a subsequent treatment, in an effort to transform such waste products into products of the alkene branched and alcoholic type, thus recovering the hydrogen and water obtained during these reactions, that will be available for their use as fuel in other chemical processing plants, or to be fed back to the gasification-pyrolysis process itself, to enrich the synthesis gas obtained.

To that effect, downstream of the gasification-pyrolysis reactor, a catalytic reactor is arranged, which operates at a temperature of 280°C, which is obtained by means of the energy obtained from the gasification-pyrolysis reactor itself, either as heat or as steam, which has previously been used to feed the gasification-pyrolysis reactor.

Inside the aforementioned catalytic reactor, the alkylating and hydration/dehydration reactions to obtain branched alkane compounds, alkenes and alcohols with a high energy content are catalysed by means of a alumina/aluminium support catalyst which acts as a Lewis acid. The temperature at which the process is carried out allows the development of the above-mentioned chemical reactions, but does not involve the deposition of solid carbon on the surface of the catalyst, since the hydrogen and water obtained as by-products of the reaction are essentially eliminated at the catalytic reactor and recycled for other uses or to be used in the gasification-pyrolysis process itself, as it has been previously mentioned, which avoids the need to carry out a cleanup of the catalysts for its use on an on-going basis, thus facilitating the production of compounds with a high energy content as a subsequent stage to the coal gasification-pyrolysis.

## Claims

1. Method related to the alkylation of residual hydrocarbons obtained from pyrolytic processes, and especially, to a process for the obtaining of synthesis gas from wet crushed coal, and essentially alkene- and alkane-type residues, so that their energy content may be exploited as isomeric and polymeric alcohols with a high energy content, **characterised in that** the waste products obtained from the formation of the synthesis gas are subject to a subsequent treatment in order to transform the said products into other branched alkane and alkene products, as well as alcoholic compounds, recovering the hydrogen and water produced in said reactions, that remain available for their subsequent use as fuel in other chemical processing plants, or to be fed back to the gasification-pyrolysis process itself, to enrich the synthesis gas obtained.

2. Method according to Claim 1, **characterised in that** the reactions used to obtain branched alkanes, alkenes and alcohols are catalysed using an aluminium-alumina-based catalyst, which acts as a Lewis acid.

3. Method for the recovery of energy from the synthesis gas waste products according to claim 1, **characterized in that** the reactions used to obtain the branched alkanes, alkenes and alcohols are carried out inside a catalytic reactor which operates at a temperature of 280°C and which is located downstream of the gasification-pyrolysis reactor.

4. Method for the recovery of energy from the synthesis gas waste products according to claim 3, **characterised in that** the said temperature is reached by feeding the energy obtained from the gasification-pyrolysis reactor itself, either as heat or as the steam previously used to feed the gasification-pyrolysis reactor.
